# EUROPÄISCHE PATENTSCHRIFT

(11) **EP 2 743 337 B1**
(45) Veröffentlichungstag und Bekanntmachung des Hinweises auf die Patenterteilung: **12.10.2016**
(21) Anmeldenummer: 12197117.0
(22) Anmeldetag: 14.12.2012
(51) Int. Cl.: C07C 303/24, C07C 305/10, A61K 8/39, A61K 8/36, A61Q 19/00, C07C 69/33, A61K 8/46

(54) **OBERFLÄCHENAKTIVE POLYGLYCERINDERIVATE**
SURFACE-ACTIVE POLYGLYCERINE DERIVATIVES
DÉRIVÉS DE POLYGLYCÉROL TENSIOACTIFS

(43) Veröffentlichungstag der Anmeldung: 18.06.2014
(73) Patentinhaber: Cognis IP Management GmbH, 40589 Dusseldorf (DE)
(72) Erfinder: Dr. Behler, Ansgar, 46240 Bottrop (DE); Clasen, Frank, 40589 Hilden (DE); Folge, Almud, 42657 Solingen (DE); Dr. Ansmann, Achim, 40699 Erkrath (DE); Dr. Kawa, Rolf, 40789 Monheim (DE); Dr. Weichold, Catherine, 52072 Aachen (DE)
(74) Vertreter: BASF IP Association

(56) Entgegenhaltungen:
- WO-A2-2010/009978
- FR-A1- 2 229 720
- US-A- 2 176 423
- US-A- 3 024 273
- TUNDO P ET AL: "The chemistry of dimethylcarbonate", ACCOUNTS OF CHEMICAL RESEARCH, ACS, WASHINGTON, DC, US, Bd. 35, 1. Januar 2002 (2002-01-01), Seiten 706-716, XP002490964, ISSN: 0001-4842, DOI: 10.1021/AR010076F [gefunden am 2002-06-05]
- HAAG R ET AL: "An approach to core-shell-type architectures in hyperbranched polyglycerols by selective chemical differentiation", MACROMOLECULES, AMERICAN CHEMICAL SOCIETY, WASHINGTON, DC; US, Bd. 33, 1. Januar 2000 (2000-01-01), Seiten 8158-8166, XP002508110, ISSN: 0024-9297, DOI: 10.1021/MA000831P [gefunden am 2000-10-07]

## Beschreibung

### Gebiet der Erfindung

Die Erfindung befindet sich auf dem Gebiet der oberflächenaktiven Verbindungen und betrifft neue nichtionische und anionische Derivate des Polyglycerins für den Einsatz unter anderem in der Kosmetik.

### Stand der Technik

Nichtionische Tenside beziehen ihre oberflächenaktiven Eigenschaften aus einem Gleichgewicht zwischen hydrophilen und hydrophoben Molekülbestandteilen. Bei den hydrophoben Anteilen handelt es sich in der Regel um aliphatische Kohlenwasserstoffreste, beispielsweise langkettige Fettsäuren oder auch Paraffine, während als hydrophile Komponenten Polyglykoletherketten, Kohlenhydrate oder Polyole in Betracht kommen.

Zu den interessantesten Polyolbausteinen, die bei der Synthese nichtionischer Tenside eingesetzt werden, zählen die Eigenkondensationsprodukte des Glycerins, die sprachlich nicht ganz zutreffend als Polyglycerine bezeichnet werden, obwohl es sich dabei um statistische Gemische von Homologen unterschiedlichen Kondensationsgrades handelt. So werden häufig auch statistische Di- und Triglycerine als Polyglycerine bezeichnet. Polyglycerine enthalten auch selten Homologe, bei denen der mittlere Kondensationsgrad oberhalb von 10 liegt, so dass in Summe eher von Oligoglycerinen gesprochen werden müsste. Dennoch hat sich in der Literatur für diese Gruppe von Verbindungen der Begriff Polyglycerine etabliert, der auch im Kontext der vorliegenden Erfindung so verstanden werden soll.

Ein typisches Merkmal nichtionischer Tenside ist das Vorhandensein eines Trübungspunktes. Hierunter versteht man einen Effekt, der auf die inverse Löslichkeit dieser Verbindungen zurückgeht oder anders ausgedrückt auf den Umstand, dass nichtionische Tenside - speziell solche, die über Polyglykoletherketten verfügen - in der Wärme schlechter löslich sind als in der Kälte, also beim Erwärmen austrüben. Grund für dieses ungewöhnliche Verhalten ist die hohe Solvatisierung der hydrophilen Ketten, die bei Erwärmen zerstört wird, d.h. in der Wärme brechen die Wasserstoffbrücken zwischen den Sauerstoffen in der hydrophilen Kette und den Wasserdipolen und die Löslichkeit nimmt ab.

Obschon Polyglycerine leicht herzustellen, preiswert, toxikologisch unbedenklich sowie stark hydrophil sind und daher ideale Ausgangsstoffe für die Synthese von Niotensiden darstellen, haben sie die teureren und in der Herstellung deutlich aufwendigeren Analogen auf Basis von Polyalkylenglykolen bislang nicht verdrängen können. Und dies selbst vor dem Hintergrund, dass die für deren Herstellung erforderlichen Ausgangsmaterialien - Ethylenoxid (EO) und Propylenoxid (PO) - petrochemisch basiert sind und somit keine nachwachsenden Rohstoffe darstellen und Herstellung und Handhabung von EO und PO wegen ihrer leichten Entzündbarkeit und hohen Toxizität keineswegs bedenkenfrei ist.

In der Praxis haben allein Ester von Polyglycerinen mit ein oder mehrwertigen Carbonsäuren eine gewisse Bedeutung erlangt. Ein Ester des Polyglycerins mit Polyhydroxystearinsäure wird beispielsweise unter der Bezeichnung Dehymuls^{®} PGPH von der BASF Personal Care & Nutrition GmbH als kosmetischer Emulgator vermarktet. Einer der Gründe für den nicht durchgreifenden Erfolg dieser Verbindungen besteht darin, dass Polyglycerine eine sehr hohe Hydroxylzahl aufweisen, also ausgesprochen hydrophil sind. Um dieses Maß an Hydrophilie auszugleichen wäre ein hoher Veresterungsgrad erforderlich, was jedoch aus sterischen Gründen schwer zu erreichen ist. Polyglycerine weisen daher in der Regel in der Regel einen Veresterungsgrad von maximal 2,2 auf. Dies führt dazu, dass Polyglycerinester mit geringerer Hydrophilie, also vergleichsweise niedrigen HLB-Werten im Bereich von etwa 10 bis 12 kaum zugänglich sind.

Aus der Internationalen Patentanmeldung WO 2010/009978 sind ethoxylierte Mono-, Di- oder Triglycerinester, insbesondere Mischungen von Mono- und Diestern bekannt, die als Lösungsvermittler in tensidhaltigen Zusammensetzungen zur Reinigung der Haut und Haare enthalten sind.

Die Aufgabe der vorliegenden Erfindung hat demnach darin bestanden, Polyglycerine in solcher Weise zu derivatisieren, dass oberflächenaktive Verbindungen erhalten werden, deren Hydrophilie gezielt eingestellt werden kann. Insbesondere sollten auch Derivate mit HLB-Werten unter 12 herstellbar sein. Insbesondere bestand die Aufgabe darin, nichtionische und/oder anionische Tenside zu entwickeln, die keine Oligo- oder Polyalkylenglykol Einheiten im Molekül enthalten (EO-frei) und einen Trübungspunkt aufweisen.

### Beschreibung der Erfindung

Gegenstand der Erfindung sind neue oberflächenaktive Polyglycerinderivate, die dadurch erhältlich sind, dass man
(a) Eigenkondensationsprodukte des Glycerins mit einem mittleren Kondensationsgrad von 1,1 bis 10 mit einem Methylierungsmittel umsetzt, so dass das Reaktionsprodukt eine Hydroxylzahl von mindestens 100 aufweist,
(b) das gebildete Salz abtrennt und die so erhaltenen Polyglycerinmethylether anschließend
(c) mit Monocarbonsäuren mit mindestens 6 Kohlenstoffatomen verestert und gegebenenfalls
(d1) die nach c) erhaltenen Polyglycerinetherester mit einem geeigneten Sulfatierungsmittel sulfatiert oder
(d2) die nach c) erhaltenen Polyglycerinetherester mit Carbonsäuren oder deren Anhydriden, die ausgewählt sind aus der Gruppe, die gebildet wird von Monocarbonsäuren, Dicarbonsäuren, Hydroxycarbonsäuren, Bernsteinsäureanhydrid sowie deren Gemischen, weiter verestert oder
(d3) die nach c) erhaltenen Polyglycerinetherester mit Maleinsäureanhydrid verestert und ggf. anschließend sulfitiert.

Vorzugsweise werden dabei solche neuen Polyglycerinderivate verestert, bei denen die Hydroxylzahl der Polglycerinmethylether, die die Komponente (b) bilden, gegenüber der Hydroxylzahl der Eigenkondensationsprodukte des Glycerins, die die Komponente (a) bilden, um mindestens 10 %, vorzugsweise um mindestens 25 % und insbesondere um mindestens 35 % vermindert ist.

Überraschenderweise wurde gefunden, dass durch Methylierung sowie nachfolgender Veresterung der Polyglycerinmethylether sowie ggf. anschließender Sulfatierung, Weiterveresterung oder Veresterung mit Maleinsäureanhydrid und anschließender Sulfitierung Derivate von Polyglycerinmethyletherester mit ausgezeichneten oberflächenaktiven Eigenschaften, insbesondere einer hohen Emulgierleistung erhalten werden. Die Methylierung , vorzugsweise unter Einsatz von Methylchlorid führt dazu, dass ein Teil der Hydroxylgruppen in den Polyglycerinen verethert werden und die Hydrophilie des Moleküls verringert wird. Die oberflächenaktiven Eigenschaften eines nichtionischen Tensids treten zu Tage, wenn die Polyglycerinmethylether im Anschluss mit Monocarbonsäuren mit mindestens 6 Kohlenstoffatomen analog c) verestert werden. Diese nichtionischen Polyglycerinetherester können in anionische Tenside überführt werden durch Derivatisierung wie der Sulfatierung nach (d1) oder Veresterung mit Maleinsäureanhydrid und ggf. anschließender Sulfitierung nach(d3). Andere oberflächenaktive nichtionische Polyglycerinderivate werden erhalten, wenn die Polyglycerinmethylester nach c) weiter verestert werden nach (d2).

Die Methylierung ist nicht mit sterischen Problemen behaftet, d.h. durch Festlegung der molaren Einsatzmenge an Methylierungsmittel wie Methylchlorid lässt sich die Hydrophilie des Polyglyceringrundkörpers vor der anschließenden Veresterung (c) und ggf. anschließender Derivatisierung (d1 bis d3) auf das gewünschte Maß einstellen. Anders ausgedrückt, lässt sich der HLB Wert für die erfindungsgemäßen Verbindungen bei gegebenem Polyglycerin regeln, indem man zuvor die Polarität des Polyglycerins durch Methylierung auf das gewünschte Maß reduziert. Verfeinert werden kann der HLB dann durch Veresterung mit der Monocarbonsäure und gegebenen Einsatzverhältnissen dieser beiden Komponenten sowie mittels der sich ggf. anschließenden Derivatisierung.

### Polyglycerine

Polyglycerine im Sinne der Erfindung stellen Eigenkondensationsprodukte des Glycerins mit einem mittleren Kondensationsgrad von 1,1 bis 10 dar, die in Gegenwart von alkalischen Verbindungen nach an sich bekannten Verfahren des Stands der Technik erhalten werden. Man erhält auf diesem Wege jedoch nur Gemische, die neben Diglycerin noch höhere Homologe sowie nicht zu vernachlässigende Mengen an nichtumgesetztem Glycerin enthalten. Einheitliche Di- und Triglycerine werden u.a. von der Firma Solvay angeboten.

Erfindungsgemäße Polyglycerinderivate mit besonders vorteilhaften Eigenschaften leiten sich vorzugsweise von statistischen Di- oder Triglycerinen ab, die aus diesen Gemischen destillativ abgetrennt werden müssen, was mit einem hohem Aufwand an Zeit und Energie verbunden ist. Alternativ lässt sich Glycerin auch in Gegenwart von Silicatverbindungen kondensieren, wie dies beispielsweise in der EP 0548104 A1 (Henkel) beschrieben wird. Das Kondensationswasser wird dabei kontinuierlich aus der Reaktionsmischung entfernt und die Reaktion abgebrochen, sobald die zur Bildung von Diglycerin theoretisch erforderliche Menge Wasser abgeschieden worden ist.

### Polyglycerinmethylether

Polyglycerinmethylether als Intermediate werden durch Umsetzung der Polyglycerine mit Methyierungsmittel, vorzugsweise Methylchlorid, erhalten.

Vorteilhafterweise werden die Polyglycerine in einem geeigneten Lösungsmittel, z. B Isopropylalkohol, gelöst, mit festen Alkalihydroxiden wie Natriumhydroxid versetzt und bei Temperaturen zwischen etwa 40 und etwa 90°C unter Druck mit Methylchlorid, das portionsweise zugesetzt wird, zur Reaktion gebracht. Nach der Reaktion wird das Produkt mit Ethanol versetzt. Dabei fällt das bei der Reaktion gebildete Natriumchlorid aus und kann abfiltriert werden. Das Lösungsmittel wird anschließend abdestilliert.

Die Aufgabe der Methylierung ist es, die Hydrophilie des Polyglycerins auf das gewünschte Maß zu reduzieren. Zu diesem Zweck werden in der Regel pro Mol Polyglycerin etwa 1 bis etwa 4 und vorzugsweise etwa 2 bis etwa 3 Mol Methylgruppen anlagert, so dass die Hydroxylzahl des resultierenden Polyglycerinmethylethers mindestens 100, vorzugsweise etwa 300 bis etwa 800 und insbesondere etwa 400 bis etwa 600 beträgt.

Aus der Eigenkondensation des Polyglycerins sind in der Regel noch Alkalirückstände enthalten, so dass man das Salz vorzugsweise als Alkalichlorid abtrennt.

### Polyglycerinmethyletherderivate

### Veresterung mit Monocarbonsäuren

In einer ersten Ausführungsform werden die Polyglycerinmethylether mit Monocarbonsäuren mit mindestens 6, vorzugsweise 6 bis 22 Kohlenstoffatomen verestert, wobei diese insbesondere ausgewählt sind aus der Gruppe der linearen oder verzweigten, gesättigten oder ungesättigten Fettsäuren mit 6 bis 22 Kohlenstoffatomen. Typische Beispiele sind Capronsäure, Caprylsäure, Caprinsäure, Isostearinsäure, Linolsäure, Linolensäure, Gadoleinsäure, Arachidonsäure, Behensäure, Erucasäure, jedoch insbesondere Laurinsäure, Myristinsäure, Palmitinsäure, Stearinsäure, Ölsäure und deren technischen Mischungen.

### Derivate der Polyglycerinmethyletherester

### Sulfate der Polyglycerinmethyletherester

In einer weiteren Ausführungsform können die Polyglycerinetherester derivatisiert werden, vorzugsweise in der Art und Weise, dass die Anzahl der nichtionischen Gruppen erhöht oder indem anionische Gruppen eingeführt werden.

Eine Art der Derivatisierung ist deren Überführung in die Sulfate mittels Sulfatierung. Als Sulfatierungsmittel, die an den noch freien Hydroxylgruppen des Polyglyceringerüstes angreifen, kommen insbesondere Schwefeltrioxid, Chlorsulfonsäure oder Amidosulfonsäure in Betracht. Nähere Ausführungen zu dem Verfahren und den Einsatzmengen sind im Zusammenhang mit dem Herstellverfahren der Sulfatderivate beschrieben.

### Ester und ggf. Sulfite der Polyglycerinmethyletherester

In einer weiteren Ausführungsform werden die Polyglycerinmethyletherester mit Carbonsäuren oder deren Anhydriden, die ausgewählt sind aus der Gruppe, die gebildet wird von Monocarbonsäuren, Dicarbonsäuren, Hydroxycarbonsäuren, Maleinsäureanhydrid und Bernsteinsäureanhydrid sowie deren Gemischen, weiter verestert.

Als Monocarbonsäuren eignen sich die bereits genannten linearen oder verzweigten, gesättigten oder ungesättigten Fettsäuren mit 6 bis 22 Kohlenstoffatomen.

Als Dicarbonsäuren bzw. Anhydride, vorzugsweise mit 2 bis 36 und insbesondere 6 bis 12 Kohlenstoffatomen, verestert, wobei diese ausgewählt sind aus der Gruppe, die gebildet wird von Oxalsäure, Malonsäure, Bernsteinsäure, Bernsteinsäureanhydrid, Glutarsäure, Adipinsäure, Pimelinsäure, Korksäure, Azelainsäure, Sebacinsäure, Dodecandisäure, Brassylsäure, Tetradecansäure, Thapsiasäure, Phthalsäure, Isophthalsäure, Terephthalsäure, Dimerfettsäure, Maleinsäure, Maleinsäureanhydrid, Fumarsäure oder deren Gemische.

Geeignete Hydroxycarbonsäuren sind aus der Gruppe Milchsäure, Citronensäure, Apfelsäure, Weinsäure, Glykolsäure, Hydroxystearinsäure und Ricinolsäure.

Bei einer weiteren Veresterung mit Maleinsäureanhydrid kann ggf. anschließend sulfitiert werden, wodurch sich Polyglycerinetheresterderivate vom Sulfosuccinat-Typ erschließen, die sich durch eine anionische Gruppe kennzeichnen.

Somit schließt die vorliegende Erfindung auch Produkte ein, die erhältlich sind, wenn Polyglycerinmethyletherester mit Maleinsäureanhydrid verestert und anschließend mit Natriumsulfit sulfitiert.

### Herstellverfahren

Ein weiterer Gegenstand der Erfindung betrifft ein Verfahren zur Herstellung von oberflächenaktiven Polyglycerinderivaten, bei dem man
a. Eigenkondensationsprodukte des Glycerins mit einem mittleren Kondensationsgrad von 1,1 bis 10 mit einem Methylierungsmittel umsetzt, so dass das Reaktionsprodukt eine Hydroxylzahl von mindestens 100 aufweist,
b. das gebildete Salz abtrennt und die so erhaltenen Polyglycerinmethylether anschließend
c. mit Monocarbonsäuren mit mindestens 6 Kohlenstoffatomen verestert und gegebenenfalls
(d1) die nach c) erhaltenen Polyglycerinetherester mit einem geeigneten Sulfatierungsmittel sulfatiert oder
(d2) die nach c) erhaltenen Polyglycerinetherester mit Carbonsäuren oder deren Anhydriden, die ausgewählt sind aus der Gruppe, die gebildet wird von Monocarbonsäuren, Dicarbonsäuren, Hydroxycarbonsäuren, Bernsteinsäureanhydrid sowie deren Gemischen, weiter verestert oder
(d3) die nach c) erhaltenen Polyglycerinetherester mit Maleinsäureanhydrid verestert und ggf. anschließend sulfitiert.

Vorzugsweise wird dabei die Hydroxylzahl der Polglycerinmethylether, die die Komponente (b) bilden, gegenüber der Hydroxylzahl der Eigenkondensationsprodukte des Glycerins, die die Komponente (a) bilden, um mindestens 10 %, vorzugsweise um mindestens 25 % und insbesondere um mindestens 35 % vermindert.

Als geeignete Methylierungsmittel kommen Methylhalogenide, insbesondere Methylchlorid in Betracht.

### Veresterung der Polyglycerinmethylether

Die Veresterung der Polyglycerinmethylether mit den Monocarbonsäuren kann in an sich bekannter Weise erfolgen; die Zugabe eines Katalysators ist in der Regel nicht erforderlich. Die Umsetzung findet vorzugsweise 100 bis 120 °C insbesondere unter Rückfluss statt. Dabei werden die Polyglycerinmethylether und die Monocarbonsäuren- bezogen auf die im Molekül enthaltenen Carboxylfunktionen - vorzugsweise im molaren Verhältnis von etwa 2:1 bis etwa 1:3,0 und insbesondere etwa 1:1,0 bis etwa 1:1,25 einsetzt. Die Veresterung kann dabei in Gegenwart organischer Lösungsmittel erfolgen. Im Anschluss an die Veresterung können die sauren Ester mit wässrigen Basen, vorzugsweise Natrium- oder Kaliumhydroxidlösung, Ammoniak oder Alkylaminen neutralisiert und in Wasser gelöst oder dispergiert werden.

### Sulfatierung der Polyglycerinmethyletherester

Zur Sulfatierung werden Sulfatierungsmittel eingesetzt, die an den freien Hydroxylgruppen des Polyglyceringerüstes angreifen. In Betracht kommen insbesondere Schwefeltrioxid, Chlorsulfonsäure oder Amidosulfonsäure. Schwefeltrioxid ist ein sehr stark oxidierendes Sulfatierungsmittel, so dass bei dessen Einsatz stets die Gefahr besteht, dass es zu Farbschädigungen des Produktes kommt. Man kann dies durch Komplexierung oder Lösemittelsulfatierung vermindern. Vorzugsweise wird daher Chlorsulfonsäure eingesetzt; alternativ kann auch Amidosulfonsäure verwendet werden. Auf diesem Wege sind jedoch keine Alkalisalze erhältlich. Die Reaktion wird wegen ihrer starken Exothermie in der Regel so geführt, dass das Sulfatierungsagens tropfenweise zudosiert und die Reaktion mit Hilfe eines Kühlmittels unter 80 °C gehalten wird. Bezogen auf die freien Hydoxylgruppen sollte die molare Einsatzmenge bei etwa 1:0,2 bis etwa 1:2,0 und vorzugsweise etwa 1:0,5 bis etwa 1:2,5 liegen.

### Veresterung der Polyglycerinmethyletherester

Die weitere Veresterung der Polyglycerinmethyletherester führt zur Einführung weiterer nichtionischer Gruppen, und damit zu einer modifizierten Oberflächenaktivität. Diese weitere Veresterung kann sowohl mit den schon beschriebenen Monocarbonsäuren, Dicarbonsäuren oder deren Anhydride in an sich bekannter Weise erfolgen; die Zugabe eines Katalysators ist in der Regel nicht erforderlich. Die Umsetzung findet vorzugsweise 100 bis 120 °C insbesondere unter Rückfluss statt. Dabei werden die Polyglycerinmethylether und die Monocarbonsäurenbezogen auf die im Molekül enthaltenen Carboxylfunktionen - vorzugsweise im molaren Verhältnis von etwa 2:1 bis etwa 1:3,0 und insbesondere etwa 1:1,0 bis etwa 1:1,25 einsetzt. Die Veresterung kann dabei in Gegenwart organischer Lösungsmittel erfolgen. Im Anschluss an die Veresterung können die sauren Ester mit wässrigen Basen, vorzugsweise Natrium- oder Kaliumhydroxidlösung, Ammoniak oder Alkylaminen neutralisiert und in Wasser gelöst oder dispergiert werden.

Im Fall der Veresterung mit Hydroxycarbonsäuren, speziell Citronensäure, kann diese jedoch auch in Abwesenheit von Lösemittel durchgeführt werden.

### Sulfitierung

Während über die Veresterung der Polyglycerinmethyletherester nichtionische Tenside erhalten werden, lassen sich anionische Tenside vom Sulfosuccinat-Typ erhalten, indem man die Veresterungsprodukte der Polyglycerinmethyletherester mit Maleinsäureanhydrid anschließend mit wässriger Alkalisulfitlösung umsetzt. Das molare Einsatzverhältnis zwischen Maleinat und Sulfit kann dabei etwa 1:0,5 bis etwa 1:1,5 betragen.

### Gewerbliche Anwendbarkeit

Die erfindungsgemäßen neuen oberflächenaktiven nichtionischen Verbindungen weisen im Gegensatz zu den nicht derivatisierten Polyglycerinen einen Trübungspunkt auf und ähneln in ihren Eigenschaften den Fettalkoholpolyglykolethern. Ein weiterer Gegenstand der vorliegenden Erfindung betrifft daher die Verwendung der neuen Polyglycerinetherderivate als Emulgatoren oder Emollients, vorzugsweise in kosmetischen Zubereitungen, in denen sie in Mengen von etwa 0,5 bis etwa 20, vorzugsweise etwa 1 bis etwa 15 und insbesondere etwa 2 bis etwa 10 Gew.-% enthalten sein können. Alternative Einsatzgebiete sind beispielsweise die Emulsionspolymerisation, die Herstellung von Lacken, Farben sowie agrochemischen Zubereitungen.

### Kosmetische Zubereitungen

Die erfindungsgemäßen neuen oberflächenaktiven Verbindungen können als Additive, speziell als Emulgatoren oder Emollients in kosmetische Zubereitungen eingesetzt werden. Diese Mittel können weitere typische Hilfs- und Zusatzstoffe enthalten, wie beispielsweise milde Tenside, Ölkörper, Co-Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, UV-Lichtschutzfaktoren, Feuchthaltemittel, biogene Wirkstoffe, Antioxidantien, Deodorantien, Antitranspirantien, Antischuppenmittel, Filmbildner, Quellmittel, Insektenrepellentien, Selbstbräuner, Tyrosininhibitoren (Depigmentie-rungsmittel), Hydrotrope, Solubilisatoren, Konservierungsmittel, Parfümöle, Farbstoffe und dergleichen enthalten.

### A. Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäure-amid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyl-lactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamid-polyglycolether, Fettaminpolyglycolether, alkoxylierte Triglyceride, Mischether bzw. Mischformale, gege-benenfalls partiell oxidierte Alk(en)yloligoglykoside bzw. Glucoron-säurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäure-sarcosinate, Fettsäuretauride, Fettsäureglutamate, α-Olefin-sulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### B. Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol^{®} CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv^{®} TN), lineare oder verzweigte, symmetrische oder unsymmetrische Dialkylether mit 6 bis 22 Kohlenstoffatomen pro Alkylgruppe, wie z.B. Dicaprylyl Ether (Cetiol^{®} OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

Als weitere Ölkörper kommen beispielsweise Silikonöle in Frage. Sie können als cyclische und/oder lineare Silikonöle vorliegen. Silikonöle sind hochmolekulare synthetische polymere Verbindungen, in denen Silicium- Atome über Sauerstoff-Atome ketten-und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel 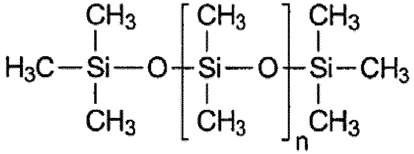 auszeichnen werden auch als Polydimethylsiloxan bzw. Dimethicon (INCI) bezeichnet. Dimethicone gibt es in verschiedenen Kettenlängen bzw. mit verschiedenen Molekulargewichten.

Vorteilhafte Polyorganosiloxane im Sinne der vorliegenden Erfindung sind beispielsweise Dimethylpolysiloxan [Poly(dimethylsiloxan)], welche beispielsweise unter den Handelsbezeichnungen Abil^{®} 10 bis 10 000 bei Evonik Goldschmidt erhältlich sind. Ferner vorteilhaft sind Phenylmethylpolysiloxan (INCI: Phenyl Dimethicon, Phenyl Trimethicon), cyclische Silikone (Octamethylcyclotetrasiloxan bzw. Decamethylcyclopentasiloxan), welche nach INCI auch als Cyclomethicon bezeichnet werden, aminomodifizierte Silikone (INCI: Amodimethicone) und Silikonwachse, z. B. Polysiloxan-Polyalkylen-Copolymere (INCI: Stearyl Dimethicon und Cetyl Dimethicon) und Dialkoxydimethylpolysiloxane (Stearoxy Dimethicon und Behenoxy Stearyl Dimethicon), welche als verschiedene Abil^{®}-Wax-Typen bei Evonik Goldschmidt erhältlich sind. Aber auch andere Silikonöle sind vorteilhaft im Sinne der vorliegenden Erfindung zu verwenden, beispielsweise Cetyidimethicon, Hexamethylcyclotrisiloxan, Polydimethylsiloxan, Poly(methylphenylsiloxan). Erfindungsgemäß besonders bevorzugte Silikone sind Dimethicon und Cyclomethicon.

### C. Emulgatoren

Als Emulgatoren kommen beispielsweise nichtionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-Polyethylenglykolalkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen^{®}-Typen (TR-1,TR-2) von Goodrich oder Cosmedia^{®} SP von Cognis;
- Polyalkylenglykole sowie
- Glycerincarbonat.

Bevorzugte Emulgatoren werden im Folgenden näher erläutert:
- **Alkoxylate.** Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.
- **Alkyl- und/oder Alkenyloligoglykoside.** Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

- **Partialglyceride.** Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.
- **Sorbitanester.** Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxy-tearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbi-tantrimaleat sowie deren technische Gemische. Ebenfalls geeignet sind Anlage-rungsprodukte von 1 bis 30, vorzugsweise 5 bis 10 Mol Ethylenoxid an die genannten Sorbitanester.
- **Anionische Emulgatoren.** Typische anionische Emulgatoren sind aliphatische Fett-säuren mit 12 bis 22 Kohlenstoffatomen, wie beispielsweise Palmitinsäure, Stearinsäure oder Behensäure, sowie Dicarbonsäuren mit 12 bis 22 Kohlenstoff-atomen, wie beispielsweise Azelainsäure oder Sebacinsäure.
- **Amphotere und kationische Emulgatoren.** Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### D. Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie Montanesterwachse, Sasolwachse, hydrierte Jojobawachse sowie synthetische Wachse, wie Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### E. Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### F. Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole^{®} und Pemulen^{®}-Typen von Goodrich; Synthalene^{®} von Sigma; Keltrol^{®}-Typen von Kelco; Sepigel^{®}-Typen von Seppic; Salcare^{®}-Typen von Allied Colloids), Polyacrylamide, Polymere, Polyvinylalkohol und Polyvinylpyrrolidon. Als besonders wirkungsvoll haben sich auch Bentonite, wie z.B. Bentone^{®} Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### G. Überfettungsmittel und Stabilisatoren

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Stabilisatoren können Metallsalze von Fettsäuren, wie z.B. Magnesium-, Aluminium- und/oder Zinkstearat bzw. -ricinoleat eingesetzt werden.

### H. Polymere

Geeignete kationische Polymere sind beispielsweise kationische Cellulosederivate, wie eine quaternierte Hydroxyethylcellulose, die unter der Bezeichnung Polymer JR 400^{®} von Amerchol erhältlich ist, kationische Stärke, Copolymere von Diallylammoniumsalzen und Acrylamiden, quaternierte Vinylpyrrolidon/Vinylimidazol-Polymere, wie Luviquat^{®} (BASF), Kondensationsprodukte von Polyglycolen und Aminen, quaternierte Kollagenpolypeptide, wie beispielsweise Lauryldimonium hydroxy-propyl hydrolyzed collagen (Lamequat^{®}L/Grünau), quaternierte Weizenpolypeptide, Polyethylenimin, kationische Siliconpolymere, wie z. B. Amidomethicone, Copolymere der Adipinsäure und Dimethylaminohydroxypropyldiethylentriamin (Cartaretine^{®}/Sandoz), Copolymere der Acrylsäure mit Dimethyldiallylammoniumchlorid (Merquat^{®} 550/Chemviron), Polyaminopolyamide sowie deren vernetzte wasserlösliche Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z. B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z. B. Mirapol^{®} A-15, Mirapol^{®} AD-1, Mirapol^{®} AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/Isobornylacrylat Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Erfindungsgemäß bevorzugt ist der Einsatz von anionischen Polymeren. Diese werden bevorzugt in Mengen von 0,1 - 5 Gew.-%, vorzugsweise 0,1 - 3 Gew.-% und insbesondere 0,1 - 2 Gew.-% bezogen auf die Gesamtzusammensetzung eingesetzt. Polyacrylsäure-Homo- und Copolymere sind erfindungsgemäß bevorzugt geeignet. Besonders vorteihafte anionische Polymere sind solche mit der INCI-Bezeichnung Carbomer wie beispielsweise Carbopole^{®} der Typen 980, 981, 1382, 2984, 5984 sowie Rheocare^{®} C plus und Rheocare^{®} 400). Weitere vorteilhafte anionische Polymere sind solche mit der INCI-Bezeichnung Acrylates/C10-30 Alkyl Acrylate Crosspolymer (z.B. Pemulen^{®} TR , Pemulen^{®} TR 2, Carbopol^{®} 1328), Acrylates Copolymer (z. B. Rheocare^{®} TTA, TTN, TTN-2), Acrylamide/Sodium Acrylate Copolymer (z.B. Cosmedia^{®} ATC), Sodium Polyacrylate (z.b. Cosmedia^{®} ATH, SP), Polyacrylamide (z.B. Sepigel^{®} 305, 501).

Als Polymere eignen sich ebenso Polysaccharide, insbesondere Xanthan-Gum, Guar-Guar, Agar-Agar, Alginate und Tylosen sowie beispielsweise Aerosil-Typen (hydrophile Kieselsäuren), Carboxymethylcellulose und Hydroxyethyl- und Hydroxypropylcellulose, Polyvinylalkohol, Polyvinylpyrrolidon und Bentonite wie z. B. Bentone^{®} Gel VS-5PC (Rheox).

Ebenso geeignet sind sogenannte quaternäre Polymere, z.B. mit der INCI - Bezeichnung Polyquaternium-37, die der folgenden allgemeinen Formel entsprechen:

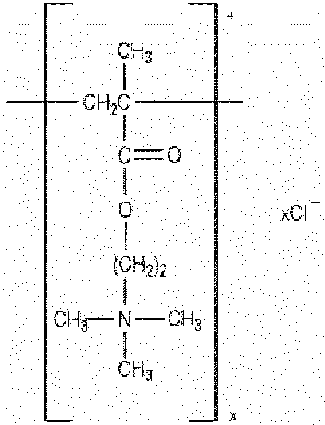

Alternativ können auch andere Dialkylaminoalkyl(meth)acrylate sowie deren durch Alkylierung oder Protonierung erhältliche Ammoniumsalze oder Dialkylaminoalkyl(meth)acrylamide sowie deren durch Alkylierung oder Protonierung erhältliche Ammoniumsalze eingesetzt werden. Besonders bevorzugt sind Polymere enthaltend MAPTAC, APTAC, MADAME, ADAME, DMAEMA und TMAEMAC. Darüber hinaus können auch Copolymere mit anionischen, weiteren kationischen oder ungeladenen Monomeren erfindungsgemäß eingesetzt werden, insbesondere solche, die neben den genannten Alkylaminoalkyl(meth)acrylat oder -(meth)acrylamid Monomeren zusätzlich (Meth)acrylsäure und/oder 2-Acrylamido-2-methyl-propansulfonsäure und/oder Acrylamid und/oder Vinylpyrrolidon und/oder Alkyl(meth)acrylate enthalten. Beispielhaft seien solche Polymere mit der INCI Bezeichnung Polyquaternium-11, Polyquaternium-13, Polyquaternium-14, Polyquaternium-15, Polyquaternium-28, Polyquaternium-32, Polyquaternium-43, Polyquaternium-47 genannt.

### I. UV-Lichtschutzfaktoren

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Üblicherweise sind die UV-Lichtschutzfaktoren in Mengen von 0,1 bis 5 und vorzugsweise 0,2 bis 1 Gew.-% zugegen. UVB-Filter können öllöslich oder wasserlöslich sein. Geeignet sind die folgenden Verbindungstypen:
1. p-Aminobenzoesäure-Derivative, zum Beispiel 4-Dimethylaminobenzoesäure 2-ethylhexyl ester;
2. Salicylsäure-Derivative, zum Beispiel Salicylsäure 2-ethylhexyl ester;
3. Benzophenon-Derivative, zum Beispiel 2-Hydroxy-4-methoxybenzophenon und seine 5-Sulfonsäure-Derivative;
4. Dibenzoylmethan-Derivative, zum Beispiel 1-(4-tert-butylphenyl)-3-(4-methoxyphenyl)propane-1,3-dione;
5. Diphenylacrylate, zum Beispiel 2-ethylhexyl 2-cyano-3,3-diphenylacrylat, und 3-(benzofuranyl) 2-cyanoacrylat;
6. 3-Imidazol-4-ylacrylsäure und deren Ester;
7. Benzofuran-Derivative, insbesondere 2-(p-aminophenyl) Benzofuranderivative, beschrieben in EP-A-582 189**,** US-A-5 338 539**,** US-A-5 518 713 and EP-A-613 893**;**
8. Polymere UV-Absorber, zum Beispiel die Benzylidenmalonat-Derivative beschrieben in EP-A-709 080**;**
9. Zimtsäurederivative, zum Beispiel 4-Methoxyzimtsäure 2-ethylhexyl ester und Isoamyl ester oder Zimtsäurederivative beschrieben in US-A-5 601 811 und WO 97/00851**;**
10. Kampherderivative, zum Beispiel 3-(4'-methyl)benzylidene-bornan-2-on, 3-benzylidenebornan-2-on, N-[2(und 4)-2-oxyborn-3-ylidene-methyl)-benzyl]acrylamide polymer, 3-(4'-trimethylammonium)-benzylidene-bornan-2-one methyl sulfate, 3,3'-(1,4-phenylene-dimethine)-bis(7,7-dimethyl-2-oxo-bicyclo[2.2.1]heptane-1-methanesulfonsäure) und Salze, 3-(4'-sulfo)benzylidene-bornan-2-on und Salze; Kampherbenzalkoniummethosulfat;
11. Hydroxyphenyltriazinverbindungen, zum Beispiel 2-(4'-methoxyphenyl)-4,6-bis(2'-hydroxy-4'-n-octyloxyphenyl)-1,3,5-triazin; 2,4-bis{[4-(3-(2-propyloxy)-2-hydroxy-propyloxy)-2-hydroxy]-phenyl}-6-(4-methoxyphenyl)-1,3,5-triazin; 2,4-bis{[4-(2-ethyl-hexyloxy)-2-hydroxy]-phenyl}-6-[4-(2-methoxyethyl-carboxyl)-phenylamino]-1,3,5-triazin; 2,4-bis{[4-(tris(trimethylsilyloxy-silylpropyloxy)-2-hydroxy]-phenyl}-6-(4-methoxyphenyl)-1,3,5-triazin; 2,4-bis{[4-(2"-methylpropenyloxy)-2-hydroxy]-phenyl}-6-(4-methoxyphenyl)-1,3,5-triazin; 2,4-bis{[4-(1',1',1',3',5',5',5'-heptamethyltrisilyl-2"-methyl-propyloxy)-2-hydroxy]-phenyl}-6-(4-methoxyphenyl)-1,3,5-triazin; 2,4-bis{[4-(3-(2-propyloxy)-2-hydroxy-propyloxy)-2-hydroxy]-phenyl}-6-[4-ethylcarboxy)-phenylamino]-1,3,5-triazin;
12. Benzotriazol Verbindungen, zum Beispiel 2,2'-methylene-bis(6-(2H-benzotriazol-2-yl)-4-(1,1,3,3-tetramethylbutyl)-phenol 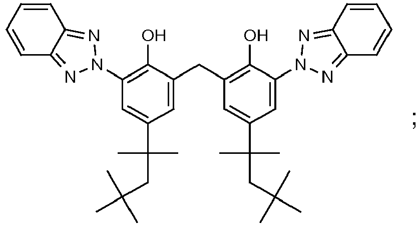
13. Trianilino-s-triazin Derivative, zum Beispiel 2,4,6-trianiline-(p-carbo-2'-ethyl-1'-oxy)-1,3,5-triazin und die UV Absorbers beschrieben in US-A-5 332 568**,** EP-A-517 104**,** EP-A-507 691**,** WO 93/17002 und EP-A-570 838**;**
14. 2-Phenylbenzimidazol-5-sulfonsäureester und deren Salze;
15. Menthyl o-aminobenzoat;
16. TiO2, ZnO und Glimmer.

Besonders bevorzugte UV-Lichtschutzmittel sind in der folgenden Tabelle aufgeführt:

| INCI | Chemical Name | CAS No. |
|---|---|---|
| 3-BENZYLIDENE CAMPHOR | 1,7,7-trimethyl-3-(phenylmethylene)bicyclo[2.2.1]heptan-2-one | 15087-24-8 |
| 4-METHYLBENZYLIDENE CAMPHOR | (+/-)-1,7,7-trimethyl-3-[(4-methylphenyl)methylene]bicyclo[2.2.1]heptan-2-one | 36861-47-9 |
| BENZOPHENONE-10 | (2-hydroxy-4-methoxyphenyl)-(4-methylphenyl)methanone | 1641-17-4 |
| BENZOPHENONE-1 | 2,4-dihydroxybenzophenone | 131-56-6 |
| BENZOPHENONE-2 | 2,2',4,4'-tetrahydroxybenzophenone | 131-55-5 |
| BENZOPHENONE-3 | 2-hydroxy-4-methoxybenzophenone | 131-57-7 |
| BENZOPHENONE-4 | 2-hydroxy-4-methoxybenzophenone-5-sulfonic acid | 4065-45-6 |
| BENZOPHENONE-6 | 2,2'-dihydroxy-4,4'-dimethoxybenzophenone | 131-54-4 |
| BENZOPHENONE-8 | 2,2'-dihydroxy-4-methoxybenzophenone | 131-53-3 |
| BENZYLIDENE CAMPHOR SULFONIC ACID | alpha-(2-oxoborn-3-ylidene)-toluene-4-sulfonic acid and its salts | 56039-58-8 |
| BUTYL METHOXYDIBEN-ZOYLMETHANE | 1-[4-(1,1-dimethylethyl)phenyl]-3-(4-methoxyphenyl)propane-1,3-dione | 70356-09-1 |
| CAMPHOR BENZALKONIUM METHOSULFATE | methyl N,N,N-trimethyl-4-[(4,7,7-trimethyl-3-oxobicyclo[2,2,1]hept-2-ylidene)methyl]anilinium sulfate | 52793-97- |
| CINOXATE | 2-ethoxyethyl p-methoxycinnamate | 104-28-9 |
| DEA-METHOXYCINNAMATE | diethanolamine salt of p-methoxyhydrocinnamate | 56265-46-4 |
| DIISOPROPYL METHYL CINNAMATE | 2-propenoic acid, 3-[2,4-bis(1-methylethyl)phenyl]-, methyl ester | 32580-71-5 |
| DIPROPYLENE GLYCOL SALICYLATE | dipropylene glycol salicylate | 7491-14-7 |
| ETHYL DIHYDROXYPROPYL PABA | ethyl 4-bis(2-hydroxypropyl)-aminobenzoate | 58882-17-0 |
| ETHYL DIISOPROPYLCIN-NAMATE | ethyl 3- [2,4-bis(1-methylethyl)phenyl]acrylate | 32580-72-6 |
| ETHYL METHOXYCINNAMATE | ethyl p-methoxycinnamate | 1929-30-2 |
| GLYCERYLOCTANOATE | | |
| DIMETHOXYCINNAMATE | | |
| GLYCERYL PABA | glyceryl 1-(4-aminobenzoate) | 136-44-7 |
| | | |
| HOMOSALATE | 3,3,5-trimethylcyclohexyl-2-hydroxybenzoate | 118-56-9 |
| ISOAMYL p-METHOXYCINNAMATE | isopentyl p-methoxycinnamate | 71617-10-2 |
| ISOPROPYL DIBENZO-YLMETHANE | 1-[4-(1-methylethyl)phenyl]-3-phenylpropane-1,3-dione | 63250-25-9 |
| ISOPROPYL METHOXYCINNAMATE | isopropyl p-methoxycinnamate | 5466-76-2 |
| LAWSONE | 2-hydroxy-1,4-naphthoquinone | 83-72-7 |
| MENTHYL ANTHRANILATE | menthyl o-aminobenzoate | 134-09-8 |
| MENTHYL SALICYLATE | menthyl salicylate | 89-46-3 |
| OCTOCRYLENE | 2-ethylhexyl 2-cyano-3,3-diphenyl acrylate | 6197-30-4 |
| ETHYLHEXYL DIMETHYL PABA | 2-ethylhexyl 4-(dimethylamino)benzoate | 21245-02-3 |
| ETHYLHEXYL METHOXYCINNAMATE | 2-ethylhexyl 4-methoxycinnamate | 5466-77-3 |
| ETHYLHEXYL SALICYLATE | 2-ethylhexyl salicylate | 118-60-5 |
| ETHYLHEXYL TRIAZONE | benzoic acid, 4,4',4"-(1,3,5-triazine-2,4,6-triyltriimino)tris-, tris(2-ethylhexyl) ester; 2,4,6-trianilino-(p-carbo-2'-ethylhexyl-1'-oxy)-1,3,5-triazine | 88122-99-0 |
| PABA | 4-aminobenzoic acid | 150-13-0 |
| PEG-25 PABA | benzoic acid, 4-amino-, ethyl ester, polymer with oxirane | 113010-52-9 |
| PENTYL DIMETHYL PABA | amyl dimethyl PABA | 14779-78-3 |
| PHENYLBENZIMIDAZOLE SULFONIC ACID | 2-phenyl-1 H-benzimidazole-5-sulfonic acid | 27503-81-7 |
| POLYACRYLAMIDOMETHYL BENZYLIDENE CAMPHOR | | 113783-61-2 |
| TEA-SALICYLATE | triethanolamine salicylate | 2174-16-5 |
| TEREPHTHALYLIDENE DICAMPHOR SULFONIC ACID | 3,3'-(1,4-phenylenedimethylene)bis[7,7-dimethyl-2-oxo-bicyclo[2.2.1]heptane-1-methanesulfonic acid] | 90457-82-2 |
| TITANIUM DIOXIDE | titanium dioxide | 13463-67-7 |
| DIGALLOYL TRIOLEATE | digalloyl trioleate | 17048-39-4 |
| ZINC OXIDE | zinc oxide | 1314-13-2 |
| Methylene bis-benzotriazolyl tetramethylbutylphenol | 2,2'-methylene-bis[6-(2H-benzotriazol-2-yl)-4-(1,1,3,3-tetramethylbutyl)-phenol] | 103597-45-1 |
| Bis-ethylhexyloxyphenol methoxyphenyltriazine | 2,4-bis{[4-(2-ethylhexyloxy)-2-hydroxy]-phenyl}-6-(4-methoxyphenyl)-(1,3,5)-triazine | 187393-00-6 |
| BISIMIDAZYLATE | 1H-benzimidazole-4,6-disulfonic acid, 2,2'-(1,4-phenylene)bis-, disodium salt | 180898-37-7 |
| DIETHYLHEXYL BUTAMIDO TRIAZONE | benzoic acid, 4,4'-[[6-[[4-[[(1,1-dimethylethyl)-amino]carbonyl]phenyl]amino]-1,3,5-triazine-2,4-diyl]diimino]bis-, bis(2-ethyl-hexyl) ester | 154702-15-5 |
| DROMETRIZOLE TRISILOX-ANE | phenol, 2-(2H-benzotriazol-2-yl)-4-methyl-6-[2-methyl-3-[1,3,3,3-tetramethyl-1-[(trimethylsilyl)oxy]disiloxanyl]propyl]- | 155633-54-8 |
| BENZYLIDENE MALONATE POLYSILOXANE | alpha-(trimethylsilyl)-omega-(trimethyl-silyloxy)poly[oxy(dimethyl)silylene]-co-[oxy(methyl)(2-{p-[2,2-bis(ethoxycarbonyl)vinyl]phenoxy}-1-methyleneethyl)silylene]-co-[oxy(methyl)(2-{p-[2,2-bis(ethoxycarbonyl)vinyl]phenoxy}prop-1-enyl)silylene] | 207574-74-1 |
| | 2-(4-diethylamino-2-hydroxybenzoyl)-benzoic hexyl ester | 302776-68-7 |

### J. Feuchthaltemittel

Feuchthaltemittel dienen zur weiteren Optimierung der sensorischen Eigenschaften der Zusammensetzung sowie zur Feuchtigkeitsregulierung der Haut. Gleichzeitig wird die Kältestabilität der erfindungsgemäßen Zubereitungen, insbesondere im Falle von Emulsionen, erhöht. Die Feuchthaltemittel sind üblicherweise in einer Menge von 0,1 bis 15 Gew.-%, vorzugsweise 1 bis 10 Gew.-%, und insbesondere 5 bis 10 Gew.-% enthalten.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole (abgekürzt = PEG) wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin, Triglycerin und Butylenglycol.

### K. Biogene Wirkstoffe und Antioxidantien

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, Alphahydroxy-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

Als Insekten-Repellentien kommen N,N-Diethyl-m-toluamid, 1,2-Pentandiol oder Ethyl Butylacetylaminopropionate in Frage. Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydro-liponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### L. Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.
- **Keimhemmende Mittel.** Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4-dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.
- **Enzyminhibitoren.** Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen^{®} CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.
- **Geruchsabsorber.** Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzyl-ethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.
   Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.
- **Antitranspirantien.** Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
   - adstringierende Wirkstoffe,
   - Ölkomponenten,
   - nichtionische Emulgatoren,
   - Coemulgatoren,
   - Konsistenzgeber,
   - Hilfsstoffe wie z. B. Verdicker oder Komplexierungsmittel und/oder
   - nichtwässrige Lösungsmittel wie z. B. Ethanol, Propylenglykol und/oder Glycerin.

   Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
   - entzündungshemmende, hautschützende oder wohlriechende ätherische Öle,
   - synthetische hautschützende Wirkstoffe und/oder
   - öllösliche Parfümöle.

   Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### M. Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### N. Antischuppenwirkstoffe

Als Antischuppenwirkstoffe kommen Pirocton Olamin (1-Hydroxy-4-methyl-6-(2,4,4-trimythylpentyl)-2-(1 H)-pyridinonmonoethanolaminsalz), Baypival^{®} (Climbazole), Ketoconazol^{®}, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol^{®}, Elubiol^{®}, Selendisulfid, Schwefel kolloidal, Schwefelpolyehtylenglykolsorbitanmonooleat, Schwefelrizinolpolyethoxylat, Schwfelteer Destillate, Salicylsäure (bzw. in Kombination mit Hexachlorophen), Undexylensäure Monoethanolamid Sulfosuccinat Na-Salz, Lamepon^{®} UD (Protein-Undecylensäurekondensat), Zinkpyrithion, Aluminiumpyrithion und Magnesiumpyrithion / Dipyrithion-Magnesiumsulfat in Frage.

### O. Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

### P. Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methylolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

### Q. Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine^{®} bekannten Silberkomplexe und die in Anlage 6, Teil A und B der Kosmetikverordnung aufgeführten weiteren Stoffklassen.

### R. Farbstoffe

Als Farbstoffe können die für kosmetische Zwecke geeigneten und zugelassenen Substanzen verwendet werden, wie sie beispielsweise in der Publikation **"**Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

### Beispiel 1

### Herstellung von Triglycerintrimethyletherestern

In einem 1-I-Rührautoklaven wurden 240,3 (1 Mol) statistisches Triglycerin, 151,5 g (3 Mol) Methylchlorid, 120 g (3 Mol) Natriumhydroxid-Prills und 300 g Isopropylalkohol bei etwa 55 °C und maximal 5 bar zur Reaktion gebracht. Nachdem der Druck bis auf einen konstanten Wert abgenommen hatte, wurde die Mischung zwei Stunden bei 90 °C nachgerührt und dann entspannt. Bezogen auf freigesetztes Chlorid wurde dabei ein Umsatz von 93,2 % der Theorie erreicht. Im Anschluss wurde das Produkt unter Zugabe von jeweils 200 ml Ethanol und anschließender Filtration dreimal gereinigt und das Lösungsmittel am Rotationsverdampfer abdestilliert. Der Triglycerintrimethylether als gelbe, klare, leicht viskose Flüssigkeit erhalten. Die Hyroxylzahl betrug 495, die Säurezahl 0,23. Der Anteil an freiem Methylchlorid wurde zu weniger als 50 ppm bestimmt, der Chloridgehalt war Null.

In einem 1-I-Dreihalskolben mit Intensivrührer und Wasserabscheider wurden 285 g (1 Mol) des oben hergestellten Triglycerintrimethylethers vorgelegt und mit jeweils 1,15 Mol verschiedener Carbonsäuren versetzt. Die Mischungen wurden unter Rückfluss erhitzt, bis kein weiteres Wasser mehr abgeschieden wurde. Anschließend wurden die Mischungen filtriert und die Trübungspunkte, Verseifungszahlen (VZ), Säurezahlen und Hydroxylzahlen (OHZ) der resultierenden Etherester bestimmt. Die Angaben dazu finden sich in Tabelle 1:

**Tabelle 1**

| Kennzahlen von Triglycerintrimethyletherestern | | | | |
|---|---|---|---|---|
| **Säurekomponente** | **Trübungspunkt (°C)** | **VZ** | **SZ** | **OHZ** |
| Laurinsäure | 41,9 | 125 | 0,5 | 159 |
| Palmfettsäure, gehärtet | 60,4 | | | |
| Stearinsäure | 45,4 | 108 | 0,6 | 146 |
| Ölsäure, technisch | 38,5 | 138 | 0,5 | 185 |

### Beispiel 2

### Herstellung von Diglycerintrimethyletherestern

Analog Beispiel 1 wurde 1 Mol statistisches Diglycerin mit 3 Mol Methylchlorid umgesetzt und der resultierende Diglycerintrimethylether von Kochsalz befreit. Anschließend wurden in einem 1-I-Dreihalskolben mit Intensivrührer und Wasserabscheider 1 Mol des Diglycerintrimethylethers vorgelegt und mit jeweils 1,15 Mol verschiedener Carbonsäuren versetzt. Die Mischungen wurden unter Rückfluss erhitzt, bis kein weiteres Wasser mehr abgeschieden wurde. Anschließend wurden die Mischungen filtriert und die Trübungspunkte, Verseifungszahlen (VZ), Säurezahlen und Hydroxylzahlen (OHZ) der resultierenden Etherester bestimmt. Die Angaben dazu finden sich in Tabelle 2:

**Tabelle 2**

| Kennzahlen von Diglycerintrimethyletherestern | | | | |
|---|---|---|---|---|
| **Säurekomponente** | **Trübungspunkt (°C)** | **VZ** | **SZ** | **OHZ** |
| Laurinsäure | 36,7 | 183 | 0,25 | 252 |
| Stearinsäure | 40,3 | 131,5 | 0,15 | 185 |
| Ölsäure, technisch | 36,7 | 183 | 0,25 | 252 |

### Beispiel 3

### Herstellung von Triglycerintrimethyletherester maleinat

In einem 1-1-Dreihalskolben mit Intensivrührer wurden 285 g (1 Mol) des Triglycerintrimethyletherester aus Beispiel 1 (Tabelle 1 erstes Beispiel) vorgelegt und mit (1,15) Mol Maleinsäureanhydrid versetzt. Die Mischungen wurden 2,5 Stunden bei 80 °C erhitzt. Das resultierende Triglycerintrimethyletherestermaleinat wurde als leicht gelblich gefärbte Lösung in praktisch quantitativer Ausbeute erhalten. Die Verseifungszahl des Produktes betrug 363, die Säurezahl 123.

### Beispiel 4

### Herstellung von Triglycerintrimethyletherestersulfosuccinat

1 Mol Triglycerintrimethyletherestermaleinat aus Beispiel 3 wurde mit 1 Mol einer 25 Gew.-%igen wässrigen Lösung von Natriumsulfit versetzt, 2 h bei 75 °C gerührt .Das resultierende Triglycerintrimethyletherestersulfosuccinat wurde als weißliche trübe Flüssigkeit in praktisch quantitativer Ausbeute erhalten. Der Wassergehalt betrug 59.9 Gew.-%, der Anteil an Waschaktiver Substanz (WAS) nach Epton 3,09 % (bestimmt als NaSO₃H).

### Beispiel 5

### Herstellung von Triglycerintrimethyletherestersuccinat

In einem 1-I-Dreihalskolben mit Intensivrührer und Wasserabscheider wurden 285 g (1 Mol) des Triglycerintrimethyletherester aus Beispiel 1 (Tabelle 1 erstes Beispiel)vorgelegt und mit (1,15) Mol Bernsteinsäureanhydrid versetzt. Die Mischungen wurden unter Rückfluss erhitzt, bis kein weiteres Wasser mehr abgeschieden wurde. Das resultierende Triglycerintrimethyletherestersuccinat wurde als leicht gelblich gefärbte Lösung in praktisch quantitativer Ausbeute erhalten.

### Beispiel 6

### Herstellung von Triglycerintrimethyletherestercitrat

In einem 1-I-Dreihalskolben mit Intensivrührer und Wasserabscheider wurden 285 g (1 Mol) des Triglycerintrimethylethers aus Beispiel 1 (Tabelle 1 erstes Beispiel) vorgelegt und mit (1,15) Mol kristalliner Citronensäure versetzt. Die Mischungen wurden unter Rückfluss erhitzt, bis kein weiteres Wasser mehr abgeschieden wurde. Das resultierende Triglycerintrimethyletherestercitrat wurde als leicht gelblich gefärbte wässrige Lösung in praktisch quantitativer Ausbeute erhalten.

### Beispiel 7

### Herstellung von Triglycerintrimethyletherestersulfat

In einem 1-I-Dreihalskolben mit Intensivrührer und Wasserabscheider wurden 285 g (1 Mol) des Triglycerintrimethylethers aus Beispiel 1 (Tabelle 1 erstes Beispiel)vorgelegt in 300 g Chloroform gelöst und tropfenweise mit (1,15) Mol Chlorsulfonsäure versetzt, wobei mit Hilfe eines Eisbads dafür gesorgt wurde, dass die Temperatur nicht über 80 °C ansteigt. Das saure Reaktionsprodukt wurde anschließend unter Zugabe von Natriummethylat (30 %-ig in Methanol) neutralisiert. Das resultierende Triglycerintrimethyletherestersulfat wurde als leicht gelblich gefärbte ölige Flüssigkeit in praktisch quantitativer Ausbeute erhalten.

## Patentansprüche

1. Oberflächenaktive Polyglycerinderivate, dadurch erhältlich, dass man (a) Eigenkondensationsprodukte des Glycerins mit einem mittleren Kondensationsgrad von 1,1 bis 10 mit einem Methylierungsmittel umsetzt, so dass das Reaktionsprodukt eine Hydroxylzahl von mindestens 100 aufweist,
(b) das gebildete Salz abtrennt und die so erhaltenen Polyglycerinmethylether anschließend
(c) mit Monocarbonsäuren mit mindestens 6 Kohlenstoffatomen verestert und gegebenenfalls
(d1) die nach c) erhaltenen Polyglycerinetherester mit einem geeigneten Sulfatierungsmittel sulfatiert oder
(d2) die nach c) erhaltenen Polyglycerinetherester mit Carbonsäuren oder deren Anhydriden, die ausgewählt sind aus der Gruppe, die gebildet wird von Monocarbonsäuren, Dicarbonsäuren, Hydroxycarbonsäuren, Bernsteinsäureanhydrid sowie deren Gemischen, weiter verestert oder
(d3) die nach c) erhaltenen Polyglycerinetherester mit Maleinsäureanhydrid verestert und ggf. anschließend sulfitiert.

2. Polyglycerinderivate nach Anspruch 1, **dadurch gekennzeichnet, dass** die Hydroxylzahl der Polglycerinmethylether, die die Komponente (b) bilden, gegenüber der Hydroxylzahl der Eigenkondensationsprodukte des Glycerins, die die Komponente (a) bilden, um mindestens 10 % vermindert ist.

3. Polyglycerinderivate nach mindestens einem der Ansprüche 1 bis 2, **dadurch gekennzeichnet, dass** es sich um statistische Di- oder Triglycerinderivate handelt.

4. Polyglycerinderivate nach mindestens einem der Ansprüche 1 bis 3, **dadurch gekennzeichnet, dass** man pro Mol Polyglycerin 1 bis 4 Mol Methylchlorid anlagert.

5. Polyglycerinderivate nach mindestens einem der Ansprüche 1 bis 4, **dadurch gekennzeichnet, dass** man als Salz Alkalichlorid abtrennt.

6. Polyglycerinderivate nach Anspruch 1, **dadurch gekennzeichnet, dass** die Monocarbonsäuren nach c) und/ oder (d2) ausgewählt sind aus der Gruppe der linearen oder verzweigten, gesättigten oder ungesättigten Fettsäuren mit 6 bis 22 Kohlenstoffatomen.

7. Polyglycerinderivate nach Anspruch 1, **dadurch gekennzeichnet, dass** die Dicarbonsäuren nach (d2) ausgewählt sind aus der Gruppe, die gebildet von Oxalsäure, Malonsäure, Bernsteinsäure, Bernsteinsäureanhydrid, Glutarsäure, Adipinsäure, Pimelinsäure, Korksäure, Azelainsäure, Sebacinsäure, Dodecandisäure, Brassylsäure, Tetradecansäure, Thapsiasäure, Phthalsäure, Isophthalsäure, Terephthalsäure, Dimerfettsäure, Maleinsäure, Maleinsäureanhydrid, Fumarsäure und deren Mischungen

8. Polyglycerinderivate nach Anspruch 1, **dadurch gekennzeichnet, dass** die Hydroxycarbonsäuren nach (d2) ausgewählt sind aus der Gruppe, die gebildet wird von Milchsäure, Citronensäure, Äpfelsäure, Weinsäure, Ricinolsäure und deren Mischungen.

9. Polyglycerinderivate nach den Ansprüchen 1 und/oder 2, **dadurch gekennzeichnet, dass** man die Polyglycerinmethyletherester nach (d3) mit Maleinsäureanhydrid verestert und anschließend mit Natriumsulfit sulfitiert.

10. Verfahren zur Herstellung von oberflächenaktiven Polyglycerinderivaten, bei dem man
(a) Eigenkondensationsprodukte des Glycerins mit einem mittleren Kondensationsgrad von 1,1 bis 10 mit einem Methylierungsmittel umsetzt, so dass das Reaktionsprodukt eine Hydroxylzahl von mindestens 100 aufweist,
(b) das gebildete Salz abtrennt und die so erhaltenen Polyglycerinmethylether anschließend
(c) mit Monocarbonsäuren mit mindestens 6 Kohlenstoffatomen verestert und gegebenenfalls
(d1) die nach c) erhaltenen Polyglycerinetherester mit einem geeigneten Sulfatierungsmittel sulfatiert oder
(d2) die nach c) erhaltenen Polyglycerinetherester mit Carbonsäuren oder deren Anhydriden, die ausgewählt sind aus der Gruppe, die gebildet wird von Monocarbonsäuren, Dicarbonsäuren, Hydroxycarbonsäuren, Bernsteinsäureanhydrid sowie deren Gemischen, weiter verestert oder
(d3) die nach c) erhaltenen Polyglycerinetherester mit Maleinsäureanhydrid verestert und ggf. anschließend sulfitiert.

11. Verfahren nach Anspruch 10, **dadurch gekennzeichnet, dass** man als Methylierungsmittel Methylchlorid einsetzt.

12. Verfahren nach Anspruch 10, **dadurch gekennzeichnet, dass** man die Polyglycerinmethylether und die Monocarbonsäuren im Verfahrensschritt (c) im molaren Verhältnis 2:1 bis 1:1,5 einsetzt.

13. Verfahren nach Anspruch 10, **dadurch gekennzeichnet, dass** man die Polyglycerinmethyletherester erhalten nach (c) mit Sulfatierungsmitteln umsetzt, die ausgewählt sind aus der Gruppe, die gebildet wird von Schwefeltrioxid, Chlorsulfonsäure oder Amidosulfonsäure.

14. Verfahren nach Anspruch 10, **dadurch gekennzeichnet, dass** man die Polyglycerinmethyletherester mit Maleinsäureanhydrid verestert und anschließend mit Natriumsulfit sulfitiert.

15. Verwendung von Polyglycerinderivaten nach Anspruch 1 als Emulgatoren oder Emollient in der Kosmetik.

## Claims

1. A surface-active polyglycerin derivative obtainable in that
(a) self-condensation products of glycerin with an average degree of condensation of 1.1 to 10 are reacted with a methylating agent such that the reaction product has a hydroxyl number of at least 100,
(b) the salt formed is separated off and then the polyglycerin methyl ethers obtained in this way
(c) are esterified with monocarboxylic acids having at least 6 carbon atoms and optionally
(d1) the polyglycerin ether esters obtained according to c) are sulfated with a suitable sulfating agent or
(d2) the polyglycerin ether esters obtained according to c) are further esterified with carboxylic acids or anhydrides thereof which are selected from the group which is formed from monocarboxylic acids, dicarboxylic acids, hydroxycarboxylic acids, succinic anhydride and mixtures thereof, or
(d3) the polyglycerin ether esters obtained according to c) are esterified with maleic anhydride and optionally then sulfited.

2. The polyglycerin derivative according to claim 1, **wherein** the hydroxyl number of the polyglycerin methyl ethers which form the component (b) is at least 10% less than the hydroxyl number of the self-condensation products of glycerin which form the component (a).

3. The polyglycerin derivative according to at least one of claims 1 to 2, **wherein** it is a random di- or triglycerol derivative.

4. The polyglycerin derivative according to at least one of claims 1 to 3, **wherein** 1 to 4 moles of methyl chloride are added per mole of polyglycerin.

5. The polyglycerin derivative according to at least one of claims 1 to 4, **wherein** alkali metal chloride is separated off as salt.

6. The polyglycerin derivative according to claim 1, **wherein** the monocarboxylic acids according to c) and/or (d2) are selected from the group of linear or branched, saturated or unsaturated fatty acids having 6 to 22 carbon atoms.

7. The polyglycerin derivative according to claim 1, wherein the dicarboxylic acids according to (d2) are selected from the group which is formed from oxalic acid, malonic acid, succinic acid, succinic anhydride, glutaric acid, adipic acid, pimelic acid, suberic acid, azelaic acid, sebacic acid, dodecanedioic acid, brassylic acid, tetradecanoic acid, thapsia acid, phthalic acid, isophthalic acid, terephthalic acid, dimer fatty acid, maleic acid, maleic anhydride, fumaric acid and mixtures thereof.

8. The polyglycerin derivative according to claim 1, **wherein** the hydroxycarboxylic acids according to (d2) are selected from the group which is formed form lactic acid, citric acid, malic acid, tartaric acid, ricinoleic acid and mixtures thereof.

9. The polyglycerin derivative according to claims 1 and/or 2, **wherein** the polyglycerine methyl ether esters according to (d3) are esterified with maleic anhydride and then sulfited with sodium sulfite.

10. A process for preparing surface-active polyglycerin derivatives, in which
(a) self-condensation products of glycerin with an average degree of condensation of 1.1 to 10 are reacted with a methylating agent such that the reaction product has a hydroxyl number of at least 100,
(b) the salt formed is separated off and then the polyglycerin methyl ethers obtained in this way
(c) are esterified with monocarboxylic acids having at least 6 carbon atoms and optionally (d1) the polyglycerin ether esters obtained according
to c) are sulfated with a suitable sulfating agent or
(d2) the polyglycerin ether esters obtained according to c) are further esterifed with carboxylic acids or anhydrides thereof which are selected from the group which is formed from monocarboxylic acids, dicarboxylic acids, hydroxycarboxylic acids, succinic anhydride and mixtures thereof, or
(d3) the polyglycerin ether esters obtained according to c) are esterified with maleic anhydride and optionally then sulfited.

11. The process according to claim 10, **wherein** methyl chloride is used as methylating agent.

12. The process according to claim 10, **wherein** the polyglycerin methyl ethers and the monocarboxylic acids in process step (c) are used in the molar ratio 2:1 to 1:1.5.

13. The process according to claim 10, **wherein** the polyglycerin methyl ether esters obtained after (c) are reacted with sulfating agents which are selected from the group which is formed form sulfur trioxide, chlorosulfonic acid or amidosulfonic acid.

14. The process according to claim 10, **wherein** the polyglycerin methyl ether esters are esterified with maleic anhydride and then sulfited with sodium sulfite.

15. The use of polyglycerin derivatives according to claim 1 as emulsifiers or emollient in cosmetics.

## Revendications

1. Dérivés tensioactifs de polyglycérol, pouvant être obtenus en ce que
(a) on transforme des produits d'autocondensation du glycérol présentant un degré moyen de condensation de 1,1 à 10 avec un agent de méthylation, de telle sorte que le produit de réaction présente un indice d'hydroxyle d'au moins 100,
(b) on sépare le sel formé et le polyglycérolméthyléther ainsi obtenu
(c) est ensuite estérifié avec des acides monocarboxyliques comprenant au moins 6 atomes de carbone et le cas échéant
(d1) on sulfate les esters de polyglycéroléther obtenus selon (c) avec un agent de sulfatation approprié ou
(d2) on estérifie davantage les esters de polyglycéroléther obtenus selon (c) avec des acides carboxyliques ou leurs anhydrides qui sont choisis dans le groupe formé par les acides monocarboxyliques, les acides dicarboxyliques, les acides hydroxycarboxyliques, l'anhydride de l'acide succinique ainsi que leurs mélanges ou
(d3) on estérifie les esters de polyglycéroléther obtenus selon (c) avec de l'anhydride de l'acide maléique et on les sulfite le cas échéant consécutivement.

2. Dérivés de polyglycérol selon la revendication 1, **caractérisés en ce que** l'indice d'hydroxyle des polyglycérolméthyléthers, qui forment le composant (b), est diminué d'au moins 10% par rapport à l'indice d'hydroxyle des produits d'autocondensation du glycérol qui forment le composant (a).

3. Dérivés de polyglycérol selon au moins l'une quelconque des revendications 1 à 2, **caractérisés en ce qu'**il s'agit de dérivés statistiques de diglycérol ou de triglycérol.

4. Dérivés de polyglycérol selon au moins l'une quelconque des revendications 1 à 3, **caractérisés en ce qu'**on additionne, par mole de polyglycérol, 1 à 4 moles de chlorure de méthyle.

5. Dérivés de polyglycérol selon au moins l'une quelconque des revendications 1 à 4, **caractérisés en ce qu'**on sépare du chlorure de métal alcalin en tant que sel.

6. Dérivés de polyglycérol selon la revendication 1, **caractérisés en ce que** les acides monocarboxyliques selon (c) et/ou selon (d2) sont choisis dans le groupe formé par les acides gras saturés ou insaturés, linéaires ou ramifiés, comprenant 6 à 22 atomes de carbone.

7. Dérivés de polyglycérol selon la revendication 1, **caractérisés en ce que** les acides dicarboxyliques selon (d2) sont choisis dans le groupe formé par l'acide oxalique, l'acide malonique, l'acide succinique, l'anhydride de l'acide succinique, l'acide glutarique, l'acide adipique, l'acide pimélique, l'acide sébacique, l'acide azélaïque, l'acide sébacique, l'acide dodécanedioïque, l'acide brassylique, l'acide tétradécanoïque, l'acide de thapsia, l'acide phtalique, l'acide isophtalique, l'acide téréphtalique, l'acide gras dimère, l'acide maléique, l'anhydride de l'acide maléique, l'acide fumarique et leurs mélanges.

8. Dérivés de polyglycérol selon la revendication 1, **caractérisés en ce que** les acides hydroxycarboxyliques selon (d2) sont choisis dans le groupe formé par l'acide lactique, l'acide citrique, l'acide malique, l'acide tartrique, l'acide ricinoléique et leurs mélanges.

9. Dérivés de polyglycérol selon les revendications 1 et/ou 2, **caractérisés en ce qu'**on estérifie les esters de polyglycérolméthyléther selon (d3) avec de l'anhydride de l'acide maléique et on les sulfite ensuite avec du sulfite de sodium.

10. Procédé pour la préparation de dérivés tensioactifs de polyglycérol, dans lequel
(a) on transforme des produits d'autocondensation du glycérol présentant un degré moyen de condensation de 1,1 à 10 avec un agent de méthylation, de telle sorte que le produit de réaction présente un indice d'hydroxyle d'au moins 100,
(b) on sépare le sel formé et le polyglycérolméthyléther ainsi obtenu
(c) est ensuite estérifié avec des acides monocarboxyliques comprenant au moins 6 atomes de carbone et le cas échéant
(d1) on sulfate les esters de polyglycéroléther obtenus selon (c) avec un agent de sulfatation approprié ou
(d2) on estérifie davantage les esters de polyglycéroléther obtenus selon (c) avec des acides carboxyliques ou leurs anhydrides qui sont choisis dans le groupe formé par les acides monocarboxyliques, les acides dicarboxyliques, les acides hydroxycarboxyliques, l'anhydride de l'acide succinique ainsi que leurs mélanges ou
(d3) on estérifie les esters de polyglycéroléther obtenus selon (c) avec de l'anhydride de l'acide maléique et on les sulfite le cas échéant consécutivement.

11. Procédé selon la revendication 10, **caractérisé en ce qu'**on utilise du chlorure de méthyle comme agent de méthylation.

12. Procédé selon la revendication 10, **caractérisé en ce qu'**on utilise le polyglycérolméthyléther et les acides monocarboxyliques dans l'étape de procédé (c) dans un rapport molaire de 2:1 à 1:1,5.

13. Procédé selon la revendication 10, **caractérisé en ce qu'**on transforme les esters de polyglycérolméthyléther obtenus selon (c) avec des agents de sulfatation qui sont choisis dans le groupe formé par le trioxyde de soufre, l'acide chlorosulfonique ou l'acide amidosulfonique.

14. Procédé selon la revendication 10, **caractérisé en ce qu'**on estérifie les esters de polyglycérolméthyléther avec de l'anhydride de l'acide maléique et on les sulfite ensuite avec du sulfite de sodium.

15. Utilisation de dérivés de polyglycérol selon la revendication 1 comme émulsifiant ou émollient en cosmétique.
